# EUROPEAN PATENT APPLICATION

(11) **EP 4 796 022 A1**
(43) Date of publication of application: **26.08.2026**
(21) Application number: 24879033.9
(22) Date of filing: 16.10.2024
(51) Int. Cl.: A61B 1/267

(54) **DISPOSABLE VIDEO LARYNGOSCOPE LENS WITH CONTAMINANT REMOVAL DEVICE**

(30) Priority: 17.10.2023 CN 202311347454; 17.10.2023 CN 202322785408 U
(71) Applicant: HADA MEDTECH INC., Markham ON L3R 9Z7 (CA)
(72) Inventor: HA, Da, Huhhot, Inner Mongolia 010050 (CN); AO, Hushan, Beijing 100037 (CN); SHU, Ni, Huhhot, Inner Mongolia 010050 (CN)
(74) Representative: AWA Sweden AB
(86) International application number: PCT/CN2024/125226
(87) International publication number: WO 2025/082392

(57) **Abstract**

Disclosed in the present invention is a disposable video laryngoscope lens with a contaminant removal device. The disposable video laryngoscope lens comprises a disposable video laryngoscope lens main body and a guide contaminant removal device, wherein the guide contaminant removal device comprises a guide tube, a pressing rod, a transmission rod and a contaminant removal rod; the guide tube is fixed to the disposable video laryngoscope lens main body, and the pressing rod and the transmission rod are inserted into the guide tube, the transmission rod is inserted and fixed on the contaminant removal rod in a split manner; and the contaminant removal rod is bent at the front end thereof to form a contaminant removal scraping rod, on which a flexible cannula is provided. The disposable video laryngoscope lens has the advantages that the contaminant removal scraping rod on the contaminant removal rod and an end surface of a high-transparency closed end of an inner cavity of the laryngoscope lens are kept parallel and move up and down under the guide action of the guide tube, such that the tip of the flexible cannula is tightly attached to the end surface of the high-transparency closed end of the inner cavity of the laryngoscope lens so as to remove contaminant. When the contaminant removal rod moves up and down under the cooperation of a guide protrusion and a corresponding guide recess, the tip of the flexible cannula is tightly attached to the end surface of the high-transparency closed end of the inner cavity of the laryngoscope lens.

## Description

### Technical Field

The present invention relates to a medical device, and in particular to a disposable video laryngoscope lens with a contaminant removal device, for use in clinical practice to lift the patient's epiglottis to expose the glottis, to guide medical personnel to perform accurate airway intubation for anesthesia or emergency rescue, and can also be used for intraoral examination and treatment.

### Background Art

The video laryngoscope was invented by Canadians in 2001. The working principle of the video laryngoscope involves disposing a camera at the front end of the laryngoscope lens; airway images are captured by the camera at the front end of the laryngoscope lens and displayed on a display screen mounted above the laryngoscope handle, allowing the operator to directly view the airway images through the display screen. The emergence of the video laryngoscope represents a revolutionary technological advance in airway management devices. The video laryngoscope provides improved glottic exposure, thereby expanding the field of view of anesthesiologists and emergency physicians and greatly improving the tracheal intubation success rate. However, even if the video laryngoscope camera is obscured by adhesive smearing from a single drop of blood or oropharyngeal secretions, the video laryngoscope immediately loses airway imaging capability, thereby critically compromising emergency rescue. At the same time, there are also methods that utilize the existing oxygen delivery line by increasing the oxygen flow rate to directly blow off adherent contaminants from the camera lens, or blow off secretions adherent to the high-transparency sealed end of the disposable inner cavity of laryngoscope lens through which the camera captures airway images and the light source passes. This contaminant removal method can only blow off loose food debris obstructing the camera lens, but is ineffective against blood or secretions adherently smeared onto the camera lens. The technical problem of video laryngoscope contaminant removal has been plaguing the industry, and there is still no ideal solution.

The applicant's prior patent CN113440092B discloses a video laryngoscope with a guide contaminant removal device, which comprises a guide tube, a contaminant removal rod, a return spring, a limit retaining ring, and the like. Although, under the guide action of the guide tube, a contaminant removal scraping rod can be closely attached to an end surface of the camera and light source at the front end of the laryngoscope lens, or to an end surface of a high-transparency closed end of an inner cavity of laryngoscope lens, for contaminant removal, it still has the following shortcomings:
1. The close attachment of the contaminant removal scraping rod at the front end of the contaminant removal rod with the end surface of the camera and light source at the front end of the laryngoscope lens, or with the end surface of the high-transparency closed end of the inner cavity of the laryngoscope lens, is achieved through a sliding fit with the guide tube. The contaminant removal rod is formed of a single unitary metal wire; therefore, when the contaminant removal rod performs reciprocated sliding displacement within the guide tube, the contaminant removal rod formed of metal wire undergoes slight elastic deformation, causing the contaminant removal scraping rod at a top end of the contaminant removal rod to undergo forward displacement, such that a rubber tube on the contaminant removal scraping rod cannot be closely attached to the end surface of the camera and light source at the front end of the laryngoscope lens or to the end surface of the high-transparency closed end of the inner cavity of the laryngoscope lens, resulting in ineffective scraping;
2. The pressing force applied to the contaminant removal rod carries a rotational component in addition to the axial downward force, causing the contaminant removal rod to rotate during reciprocated sliding displacement within the guide tube; when the contaminant removal rod performs reciprocated displacement, the front end of the contaminant removal rod undergoes outward rotational deflection, causing frictional interference between the contaminant removal rod within the limiting cavity and the limit retaining ring or an outer wall of the laryngoscope lens, resulting in binding and sticking of the contaminant removal rod during pressing actuation; at the same time, due to the rotation of the contaminant removal rod, the rubber tube on the contaminant removal scraping rod at the top end of the contaminant removal rod cannot be closely attached to the end surface of the camera and light source at the front end of the laryngoscope lens or to the end surface of the high-transparency closed end of the inner cavity of the laryngoscope lens, resulting in ineffective scraping;
3. The contaminant removal rod is formed of a single unitary metal wire. The contaminant removal rod on adult laryngoscopes is typically approximately 130mm in length. During the mass production process, the contaminant removal rod must be bent at multiple angles to conform to the curved profile of the curved-type laryngoscope lens, resulting in complex manufacturing processes, difficulty in controlling dimensional precision, low production yield in mass industrial manufacturing, low production efficiency, and high production costs.

### Summary of the Invention

In order to overcome the aforementioned technical deficiencies of the video laryngoscope, it is an object of the present invention to provide a guide contaminant removal device such that a flexible cannula or flexible scraping strip on a contaminant removal rod is closely attached to an end surface of a high-transparency closed end, and the contaminant removal rod and the contaminant removal rod are kept parallel and move up and down through a return spring, thereby effectively scraping off blood and secretions adherently smeared on the airway image capture region of the camera or video laryngoscope lens, enabling real-time airway image capture by the camera and ensuring normal operation of the video laryngoscope - the present invention being a disposable video laryngoscope lens with a contaminant removal device.

The invention discloses a disposable video laryngoscope lens with a contaminant removal device, comprising a disposable video laryngoscope lens main body. The disposable video laryngoscope lens main body comprises a high-transparency closed end of an inner cavity of laryngoscope lens for image capture by a camera and passage of a light source. The disposable video laryngoscope lens further comprises a guide contaminant removal device, the guide contaminant removal device comprising a guide tube, a pressing rod, a transmission rod, and a contaminant removal rod. The guide tube is fixed on an outer wall of a tail end of the disposable video laryngoscope lens main body. The axial direction of the guide tube is parallel to the end surface of the high-transparency closed end of the inner cavity of laryngoscope lens. One end of the pressing rod and one end of the transmission rod are slidably inserted into the guide tube; a pressing head is disposed on a top end of the pressing rod at an outer side of the guide tube; a tip of the transmission rod at the outer side of the guide tube is inserted and fixed to the contaminant removal rod in a split manner; A guide protrusion is disposed on an outer wall of the disposable video laryngoscope lens main body, and a length direction of the guide protrusion is parallel to the end surface of the high-transparency closed end of the inner cavity of laryngoscope lens; the contaminant removal rod is provided with a guide recess corresponding to the guide protrusion, the guide recess is slidably snap-fitted onto the guide protrusion, and the contaminant removal rod performs reciprocated displacement on the outer wall of the disposable video laryngoscope lens main body along the axial direction of the guide tube through the guide recess slidably snap-fitted onto the guide protrusion; the contaminant removal rod is bent at the front end thereof to form a contaminant removal scraping rod, on which a flexible cannula or flexible scraping strip that is closely attached to the end surface of the high-transparency closed end of the inner cavity of the laryngoscope lens is provided.

Preferably, a cannula seat is fixedly disposed on the outer wall of the tail end of the disposable video laryngoscope lens main body, above the guide protrusion, and the guide tube is inserted and fixed within the cannula seat in a split manner.

Preferably, the guide tube is a concentric reducing tube, and the inner cavity of the guide tube is divided into a pressing rod cavity and a transmission rod cavity. A pipe diameter of the pressing rod cavity is greater than a pipe diameter of the transmission rod cavity. The pressing rod is disposed in the pressing rod cavity along the axial direction of the guide tube in a reciprocated sliding manner. The transmission rod is disposed in the transmission rod cavity in a reciprocated sliding manner along the axial direction of the guide tube. One end of the transmission rod is inserted and fixed to one end of the pressing rod within the pressing rod cavity in a split manner. A return spring is sleeved onto the transmission rod within the pressing rod cavity.

Preferably, the flexible cannula has a cross section in a shape of water drop, and the tip of the flexible cannula is closely attached to the end surface of the high-transparency closed end of the inner cavity of laryngoscope lens.

Preferably, the disposable video laryngoscope lens main body comprises the inner cavity of laryngoscope lens and a tongue depressor, the rear end of a cavity body of the inner cavity of the laryngoscope lens is open, and the inner cavity of the laryngoscope lens is inserted onto an extension arm.

Advantages of the present invention: in the present application, the contaminant removal scraping rod on the contaminant removal rod and the end surface of the high-transparency closed end of the inner cavity of the laryngoscope lens are kept parallel and move up and down under the guide action of the guide tube, such that the tip of the flexible cannula is tightly attached to the end surface of the high-transparency closed end of the inner cavity of the laryngoscope lens so as to remove contaminant. By pressing the contaminant removal rod with the thumb to move up and down, thumb actuation is unrestricted, the operation is convenient and effort-saving, and the stability of the laryngoscope during handheld operation is not affected. During reciprocated displacement of the contaminant removal rod under the cooperation of the guide protrusion and the corresponding guide recess, the tip of the flexible cannula is tightly attached to the end surface of the high-transparency closed end of the inner cavity of the laryngoscope lens.

### Brief Description of the Drawings

FIG. 1 is an overall schematic view of the present invention.
FIG. 2 is a schematic view illustrating use of the present invention.
FIG. 3 is a sectional view of a partial structure of the present invention.
FIG. 4 is a sectional view of a guide tube.
FIG. 5 is a sectional view of a pressing rod cavity.
FIG. 6 is an oblique view of FIG. 1.
FIG. 7 is a sectional view of a cross section of a contaminant removal scraping rod.

### Detailed Description of Embodiments

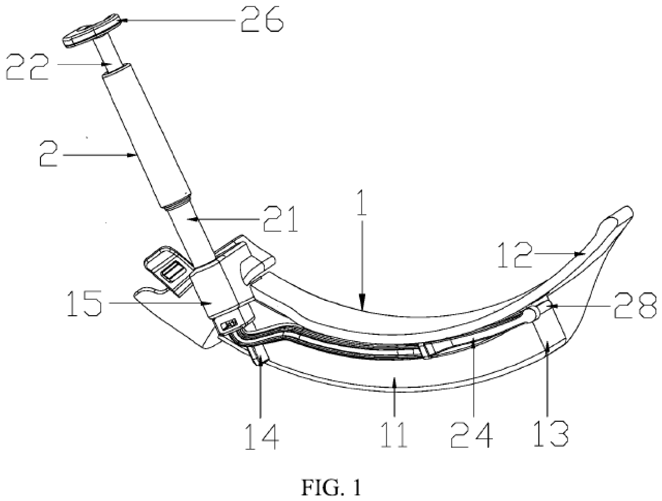

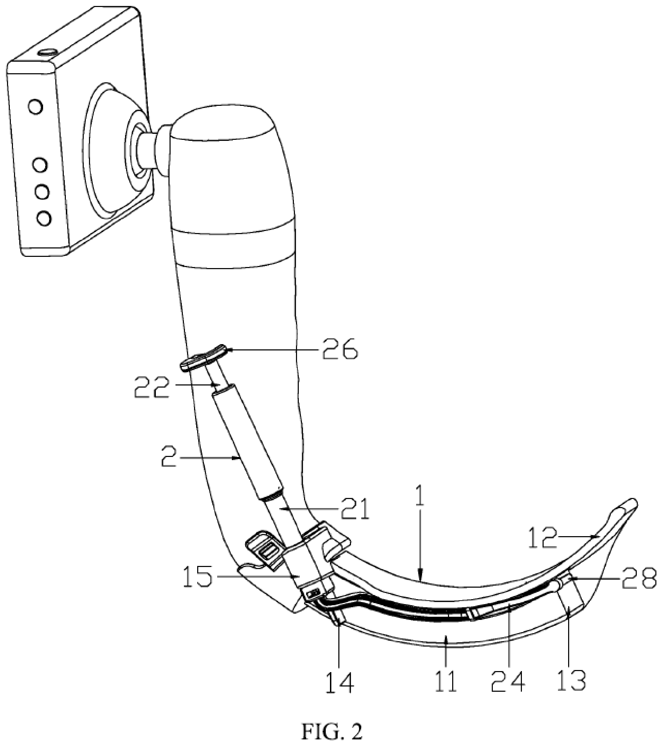

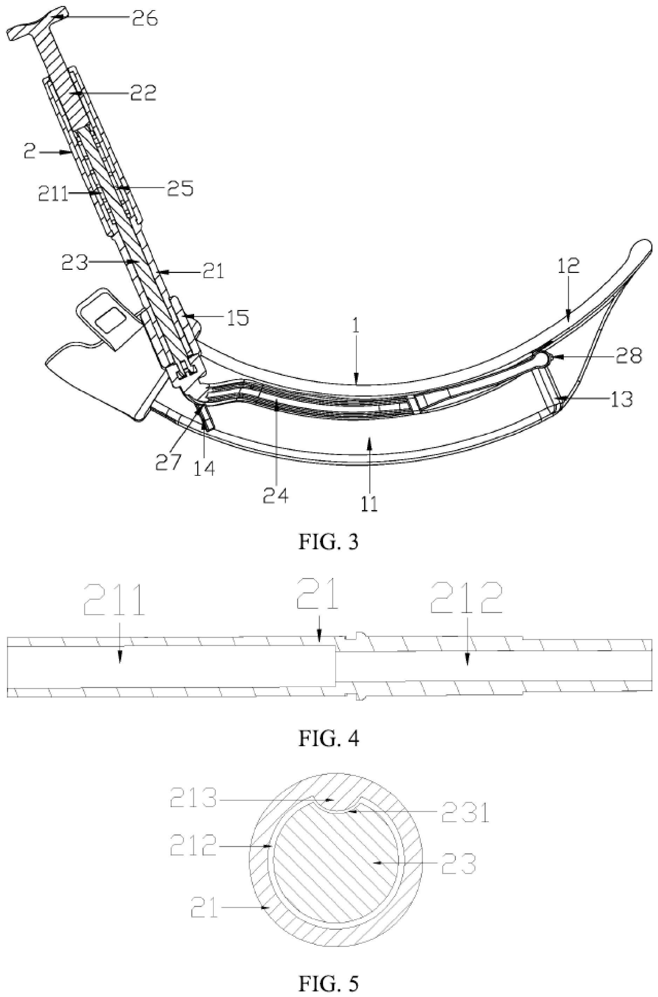

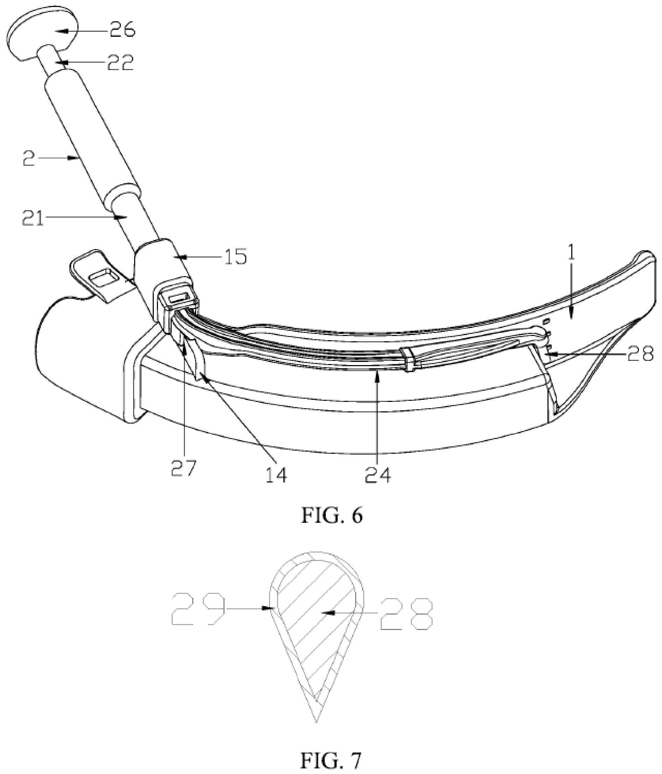

**Embodiment 1:** As shown in FIGS. 1 to 3, a disposable video laryngoscope lens with a contaminant removal device comprises a disposable video laryngoscope lens main body 1 and a guide contaminant removal device 2. The disposable video laryngoscope lens main body 1 comprises an inner cavity 11 of the laryngoscope lens; a tongue depressor 12, wherein a rear end of a cavity body of the inner cavity 11 of the laryngoscope lens is open; and a high-transparency closed end 13 of the inner cavity of the laryngoscope lens used for image capture by a camera and passage of a light source. A guide protrusion 14 is disposed on an outer wall of the disposable video laryngoscope lens main body 1. A length direction of the guide protrusion 14 is parallel to an end surface of the high-transparency closed end 13 of the inner cavity of the laryngoscope lens; a cannula seat 15 is fixedly disposed on an outer wall of the tail end of the disposable video laryngoscope lens main body 1 above the guide protrusion 14. The guide contaminant removal device 2 comprises a guide tube 21, a pressing rod 22, a transmission rod 23, and a contaminant removal rod 24. The guide tube 21 is inserted and fixed within the cannula seat 15 in a split manner. An axial direction of the guide tube 21 is parallel to the end surface of the high-transparency closed end 13 of the inner cavity of the laryngoscope lens; as shown in FIG. 4, the guide tube 21 is a concentric reducing tube. An inner cavity of the guide tube 21 is divided into a pressing rod cavity 211 and a transmission rod cavity 212. A pipe diameter of the pressing rod cavity 211 is greater than a pipe diameter of the transmission rod cavity 212. The pressing rod 22 is disposed in the pressing rod cavity 211 in a reciprocated sliding manner along the axial direction of the guide tube 21. The transmission rod 23 is disposed in the transmission rod cavity 212 in a reciprocated sliding manner along the axial direction of the guide tube 21; among them, as shown in FIG. 5, a guide key 213 is disposed on the inner wall of the transmission rod cavity 212 along the axial direction of the guide tube 21, and the outer wall of the transmission rod 23 is provided with a mated guide recess 231 corresponding to the guide key 213; by means of the guide recess 231 correspondingly mated with the guide key 213, the transmission rod 23 is prevented from rotating during reciprocated sliding within the guide tube 21 along the axial direction of the guide tube 21; one end of the transmission rod 23 is inserted and fixed to one end of the pressing rod 22 within the pressing rod cavity 211 in a split manner, and a return spring 25 is sleeved onto the transmission rod 23 within the pressing rod cavity 211; a pressing head 26 is disposed on a top end of the pressing rod 22 at an outer side of the guide tube 21; a tip of the transmission rod 23 at the outer side of the guide tube 21 is inserted and fixed on the contaminant removal rod 24 in a split manner; as shown in FIG. 6, the contaminant removal rod 24 is provided with a guide recess 27 corresponding to the guide protrusion 14; the guide recess 27 is slidably snap-fitted onto the guide protrusion 14; the contaminant removal rod 2 performs reciprocated displacement on the outer wall of the disposable video laryngoscope lens main body 1 along the axial direction of the guide tube 21 through the guide recess 27 slidably snap-fitted onto the guide protrusion 14; by means of the sliding fit between the guide protrusion 14 and the guide recess 27, extending the guided sliding length of the reciprocated displacement of the contaminant removal rod 24, such that the contaminant removal rod 24 is prevented from exhibiting axial play along the disposable video laryngoscope lens main body 1 or lateral oscillation during reciprocated displacement, ensuring stable and precise positioning of the contaminant removal rod 24 throughout reciprocated displacement; the contaminant removal rod 24 is bent at the front end thereof to form a contaminant removal scraping rod 28; the contaminant removal scraping rod 28 is disposed with a flexible cannula 29 that is closely attached to the end surface of the high-transparency closed end 13 of the inner cavity of the laryngoscope lens; as shown in FIG. 7, the flexible cannula 29 has a cross section in a shape of water drop, and a tip of the flexible cannula 29 having the cross section in the shape of water drop is closely attached to the end surface of the high-transparency closed end 13 of the inner cavity of the laryngoscope lens.

During use, the disposable video laryngoscope lens main body 1 is removably inserted onto the extension arm. During the procedure, when secretions become adherently deposited on the high-transparency closed end 13 of the inner cavity of laryngoscope lens, the operator presses the pressing head 26, causing the pressing rod 22, the transmission rod 23, and the contaminant removal rod 24 to operate in coordinated linkage; the tip of the flexible cannula 29 on the contaminant removal scraping rod 28 scrapes off secretions adherent to the end surface of the high-transparency closed end 13 of the inner cavity of laryngoscope lens, enabling the camera to capture airway images in real time.

## Claims

1. A disposable video laryngoscope lens with a contaminant removal device, comprising a disposable video laryngoscope lens main body, the disposable video laryngoscope lens main body comprising a high-transparency closed end of an inner cavity of the laryngoscope lens for image capture by a camera and passage of a light source, wherein the disposable video laryngoscope lens further comprises a guide contaminant removal device, the guide contaminant removal device comprises a guide tube, a pressing rod, a transmission rod, and a contaminant removal rod, the guide tube is fixed on an outer wall of a tail end of the disposable video laryngoscope lens main body, an axial direction of the guide tube is parallel to an end surface of the high-transparency closed end of the inner cavity of the laryngoscope lens, and one end of the pressing rod and one end of the transmission rod are slidably inserted into the guide tube; a pressing head is disposed on a top end of the pressing rod at an outer side of the guide tube; a tip of the transmission rod at the outer side of the guide tube is inserted and fixed to the contaminant removal rod in a split manner; a guide protrusion is disposed on the outer wall of the disposable video laryngoscope lens main body, and a length direction of the guide protrusion is parallel to an end surface of the high-transparency closed end of the inner cavity of the laryngoscope lens; the contaminant removal rod is provided with a guide recess corresponding to the guide protrusion, the guide recess is slidably snap-fitted onto the guide protrusion, and the contaminant removal rod performs reciprocated displacement on the outer wall of the disposable video laryngoscope lens main body along the axial direction of the guide tube through the guide recess slidably snap-fitted onto the guide protrusion; the contaminant removal rod is bent at the front end thereof to form a contaminant removal scraping rod, on which a flexible cannula or flexible scraping strip that is closely attached to the end surface of the high-transparency closed end of the inner cavity of the laryngoscope lens is provided.

2. The disposable video laryngoscope lens with a contaminant removal device according to claim 1, wherein a cannula seat is fixedly disposed on the outer wall of the tail end of the disposable video laryngoscope lens main body above the guide protrusion, and the guide tube is inserted and fixed within the cannula seat in a split manner.

3. The disposable video laryngoscope lens with a contaminant removal device according to any one of claim 1 or 2, wherein the guide tube is a concentric reducing tube, an inner cavity of the guide tube is divided into a pressing rod cavity and a transmission rod cavity, a pipe diameter of the pressing rod cavity is greater than a pipe diameter of the transmission rod cavity, the pressing rod is disposed in the pressing rod cavity in a reciprocated sliding manner along the axial direction of the guide tube, the transmission rod is disposed in the transmission rod cavity in a reciprocated sliding manner along the axial direction of the guide tube, one end of the transmission rod is inserted and fixed to one end of the pressing rod within the pressing rod cavity in a split manner; and a return spring is sleeved onto the transmission rod within the pressing rod cavity.

4. The disposable video laryngoscope lens with a contaminant removal device according to claim 1, wherein the flexible cannula has a cross section in a shape of water drop, and a tip of the flexible cannula having the cross section in the shape of water drop is closely attached to the end surface of the high-transparency closed end of the inner cavity of the laryngoscope lens.

5. The disposable video laryngoscope lens with a contaminant removal device according to claim 1, wherein the disposable video laryngoscope lens main body comprises the inner cavity of the laryngoscope lens and a tongue depressor, a rear end of a cavity body of the inner cavity of the laryngoscope lens is open, and the inner cavity of the laryngoscope lens is inserted onto an extension arm.
